# FASCICULE DE BREVET EUROPEEN

(11) **EP 0 575 481 B1**
(45) Date de publication et mention de la délivrance du brevet: **29.05.1996**
(21) Numéro de dépôt: 92907934.1
(22) Date de dépôt: 10.03.1992
(51) Int. Cl.: A61K 31/445, A61K 9/00, A61K 47/10

(54) **NOUVELLES COMPOSITIONS LIQUIDES A BASE DE DERIVES DE LA PIPERIDINE SUBSTITUES EN 1,4**
NEUE FLÜSSIGE ZUSAMMENSETZUNGEN AUF BASIS VON 1,4-SUBSTITUIERTEN PIPERIDIN-DERIVATEN
NOVEL LIQUID COMPOSITIONS BASED ON DERIVATIVES OF 1,4 SUBSTITUTED PIPERIDINE

(30) Priorité: 13.03.1991 FR 9103049
(43) Date de publication de la demande: 29.12.1993
(73) Titulaire: RHONE-POULENC RORER S.A., 92160 Antony (FR)
(72) Inventeur: BOBEE, Jean-Marc, F-91370 Verrières-le-Buisson (FR); COUTEL, Anne, F-91120 Palaiseau (FR)
(74) Mandataire: Le Pennec, Magali
(86) Numéro de dépôt international: FR9200215
(87) Numéro de publication internationale: WO9216210

(56) Documents cités:
- EP-A- 0 134 124
- WO-A-88/08302
- WO-A-88/09656
- WO-A-89/10143
- RÖMPP CHEMIE LEXIKON, 9. Auflage, 1990 "Lösungsvermittler"
- RÖMPP CHEMIE LEXIKON, 9. Auflage, 1992 "Polyethylenglykole"

## Description

La présente invention concerne une nouvelle forme pharmaceutique à base de dérivés de la piperidine substitués en 1,4.

Les composés pharmaceutiques objet de la formulation décrite dans la présente invention sont inclus dans la demande de brevet européen EP 134 124.

Ces composés et encore plus spécifiquement le composé suivant dont la dénomination internationale est l'ébastine ou 4-diphénylméthoxy 1-[3-(4-ter-butylbenzoyl)propyl] piperidine de formule: 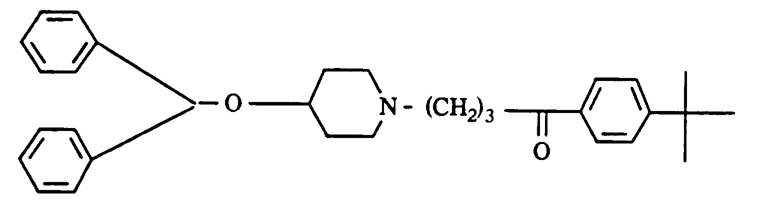
ne sont pas solubles dans l'eau. Il est ainsi difficile de les mettre sous une forme pharmaceutique aqueuse telle qu'un sirop.

Le sel formé entre le composé ci-dessus et l'acide lactique présente son optimum de solubilité à pH 2, solubilité qui est tout de même limitée à 0,8 mg/ml, mais il ne présente une stabilité optimale qu'à pH 4. Ainsi les pH de solubilité optimale et de stabilité optimale ne peuvent en aucun cas permettre d'obtenir des solutions stables d'ébastine ayant une concentration minimale de 1 mg/ml.

Ces composés ont une activité antihistaminique H1 et sont utiles dans le traitement des maladies respiratoires, allergiques ou cardiovasculaires. Ils relâchent ainsi in vitro et in vivo les muscles lisses vasculaires et bronchiques.

Ils inhibent aussi l'effet constricteur de l'adrénaline, des ions potassium tant au niveau intestinal qu'au niveau de la trachée. Ils bloquent ainsi la bronchoconstriction provoquée par les aérosols d'histamine à des doses aussi faibles que 1 mg/kg.

Ces composes sont actifs par voie parentérale ainsi que par voie orale. Lors de leur administration par voie orale il est nécessaire d'utiliser un sel de l'acide carboxylique dont la solubilité dans l'eau est toujours faible. Ainsi dans la demande de brevet préalablement citée c'est à dire la demande de brevet européen EP 134 124, exemple 7, il a été nécessaire pour former une solution d'ajouter au principe actif préalablement décrit sous sa forme acide, un émulsifiant tel que l'huile de ricin hydrogénée et éthoxylée plus connue sous la dénomination commerciale de Crémophor à des doses loin d'être négligeables puisque comprises aux environs de 2 % en poids par rapport à la solution finale, ce qui représente une quantité d'émulsifiant égale à quatre fois la quantité de principe actif.

Or, il est toujours difficile dans l'industrie pharmaceutique de proposer sur le marché une solution qui se présente plus sous l'aspect d'une solution savonneuse que d'un sirop. Nous avons donc cherché à éviter l'usage de tout émulsifiant dans notre formulation. L'émulsifiant utilisé dans la composition du brevet EP 134 124 a en plus deux inconvénients importants il présente un goût ne permettant pas d'obtenir une aromatisation satisfaisante et provoque des phénomènes d'intolérance conduisant au rejet du médicament.

La présente invention concerne une nouvelle composition pharmaceutique liquide aqueuse, exempte d'agent tensioactif caractérisé en ce qu'elle contient à titre de principe actif un composé répondant à la formule : 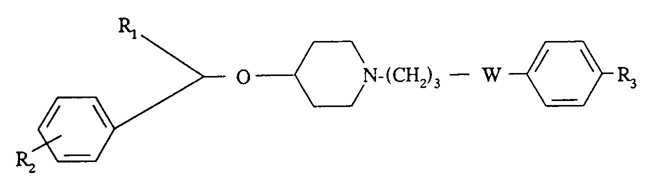
dans laquelle :
- R₁ représente un groupe thiényl, un groupe phényl éventuellement substitué par un halogène, un groupe alcoxy contenant 1 à 6 atomes de carbone, un groupe alkyle contenant 1 à 6 atomes de carbone,
- R₂ représente un atome d'halogène, un atome d'hydrogène, un groupe alcoxy contenant 1 à 6 atomes de carbone, un groupe alkyle contenant 1 à 6 atomes de carbone,
- R₃ représente un atome d'halogène, un atome d'hydrogène, un groupe alcoxy contenant 1 à 6 atomes de carbone, un groupe alkyle contenant 1 à 6 atomes de carbone, un groupe alkylthio contenant 1 à 6 atomes de carbone, un groupe cycloalkyle contenant 5 ou 6 atomes de carbone ou un groupe de formule : 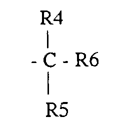 où R4 et R5 représentent indépendamment l'un de l'autre un atome d'hydrogène ou un groupe alkyle contenant 1 à 6 atomes de carbone, R6 représente un groupe cycloalkyle contenant 3 à 6 atomes de carbone, hydroxyméthyl, carboxy ou alcoxycarbonyle contenant 2 à 7 atomes de carbone,
- W représente un groupe carbonyle ou hydroxyméthylène, ou leurs sels, ladite composition contenant du polyéthylèneglycol à titre d'agent solubilisant.

Cette composition est caractérisée par le fait que l'agent solubilisant du principe actif est de préférence le polyéthylène glycol 600.

Cette composition présente l'avantage de ne pas nécessiter la présence d'agent conservateur et s'aromatise beaucoup plus aisément que la solution contenant l'agent tensioactif.

Cette composition contient une quantité de polyéthylène glycol telle que le rapport pondéral entre le polyéthylène glycol et le principe actif est compris, de préférence entre 100 et 500 et encore plus préférentiellement entre 100 et 200.

La composition de la présente invention présente les avantages suivants par rapport à la composition de l'exemple 7 du brevet préalablement mentionné :
- elle ne présente plus ni aspect ni goût savonneux
- elle ne nécessite plus la présence d'agents de conservation tels que les parahydroxybenzoates d'alkyle
- elle est facilement aromatisable
- elle présente une bonne conservation sans aucune séparation de particules contrairement à ce qui apparait pour la solution de l'exemple 7.

La composition est de préférence maintenue à un pH compris entre 4 et 4,5 qui est son pH optimum de stabilité par addition d'acide lactique.

La composition sous forme de sirop préférée selon l'invention contient:

| | |
|---|---|
| - ébastine | 0,1 % |
| - polyéthylène glycol 600 | 16 % |
| - édulcorant | 60 % |
| - acide lactique | q.s.p. pH 4,2 |
| - eau | q.s.p. 100 % |

On préfère utiliser comme édulcorant le Lycasin® ou sirop de glucose hydrogéné, en raison de ses propriétés non cariogènes.

Cette composition est facilement aromatisable. Elle peut être utilisée chez l'enfant sans risque de rejet et sans dégoût, pour le traitement des manifestations allergiques surtout respiratoires.

La présente invention sera plus complètement décrite à l'aide des exemples suivants qui ne doivent pas être considérés comme limitatifs de l'invention.

### EXEMPLE 1

Pour la préparation de cette composition on met en oeuvre les produits suivants:

| | |
|---|---|
| Ebastine | 0,100 g |
| Polyéthylène glycol 600 | 16,000 g |
| Lycasin® 80/55 (sirop de glucose hydrogéné) | 60,000 g |
| Acide lactique N q.s.p. | pH 4,2 0,624 g |
| Arôme | 1,000 g |
| Eau quantité suffisante pour | 100,000 ml |

On mélange pendant 30 minutes à chaud (entre 40 et 60°C) et sous agitation l'Ebastine, le polyéthylène glycol et l'acide lactique. On ajoute au mélange précédent 60% de l'eau, on agite pour homogénéiser puis on ajoute le Lycasin®.

On vérifie le pH qui est à 4,2. On ajoute le complément d'eau.

Cette composition se présente sous une forme liquide qui ne présente aucun dépôt après 6 mois de conservation.

### EXEMPLE COMPARATIF SELON LE BREVET EP 134 124

On met en oeuvre :

| | |
|---|---|
| Ebastine | 1 g |
| Acide lactique | 8,3 g |
| Glycerine | 6 g |
| Huile de ricin hydrogénée et éthoxylée | 4 g |
| Méthyl p-hydroxybenzoate de sodium | 0,32 g |
| Propyl p-hydroxybenzoate de sodium | 0,08 g |
| Saccharinate de sodium | 0,4 g |
| Arôme | |
| Hydroxide de sodium | q.s.p. pH=4 |
| Eau | q.s.p. 200 ml |

A une solution de méthyl et propyl p-hydroxybenzoates de sodium et de saccharinate de sodium, dans 20 ml d'eau, on ajoute une solution aqueuse d'acide lactique et d'huile de ricin hydrogénée et éthoxylée. Après agitation, on ajoute l'Ebastine et on homogénéise pour obtenir une complète dissolution. Après cela, on mélange l'agent d'aromatisation sous agitation vigoureuse, puis on complète au volume final avec l'eau.

La solution obtenue présente un goût de savon très prononcé et précipite après 24 heures de conservation.

## Revendications

1. Composition pharmaceutique liquide aqueuse, exempte d'agent tensioactif caractérisé en ce qu'elle contient à titre de principe actif un composé répondant à la formule : 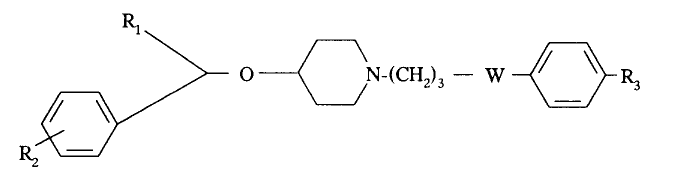 dans laquelle :
- R₁ représente un groupe thiényl, un groupe phényl éventuellement substitué par un halogène, un groupe alcoxy contenant 1 à 6 atomes de carbone, un groupe alkyle contenant 1 à 6 atomes de carbone,
- R₂ représente un atome d'halogène, un atome d'hydrogène, un groupe alcoxy contenant 1 à 6 atomes de carbone, un groupe alkyle contenant 1 à 6 atomes de carbone,
- R₃ représente un atome d'halogène, un atome d'hydrogène, un groupe alcoxy contenant 1 à 6 atomes de carbone, un groupe alkyle contenant 1 à 6 atomes de carbone, un groupe alkylthio contenant 1 à 6 atomes de carbone, un groupe cycloalkyle contenant 5 ou 6 atomes de carbone ou un groupe de formule : 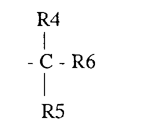 où R4 et R5 représentent indépendamment l'un de l'autre un atome d'hydrogène ou un groupe alkyle contenant 1 à 6 atomes de carbone, R6 représente un groupe cycloalkyle contenant 3 à 6 atomes de carbone, hydroxyméthyl, carboxy ou alcoxycarbonyle contenant 2 à 7 atomes de carbone,
- W représente un groupe carbonyle ou hydroxyméthylène, ou leurs sels, ladite composition contenant du polyéthylèneglycol à titre d'agent solubilisant.

2. Composition selon la revendication 1 caractérisée en ce que le principe actif est défini comme dans la revendication 1, pourvu que :
- R1 représente un groupe phényle
- R2 représente l'hydrogène
- R3 représente un groupe tertiobutyle
- W représente un groupe carbonyl.

3. Composition selon la revendication 1 ou 2 caractérisée en ce qu'elle contient un rapport pondéral polyéthylène glycol/composé selon la revendication 1 compris entre 100 et 500 et de préférence compris entre 100 et 200.

4. Composition selon l'une quelconque des revendications précédentes caractérisée en ce qu'elle contient :
- 0,1 % de composé selon la revendication 1
- de l'acide lactique en quantité suffisante pour ajuster le pH à environ 4,2
- 16 % de polyéthylène glycol
- 60 % d'agent édulcorant
et dont le complément à 100 % est de l'eau.

## Claims

1. Aqueous liquid pharmaceutical composition free from surfactant, characterized in that it contains as active principle a compound corresponding to the formula: 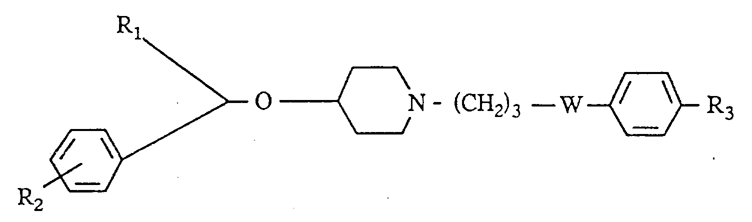 in which:
- R₁ represents a thienyl group, a phenyl group optionally substituted with a halogen, an alkoxy group containing 1 to 6 carbon atoms or an alkyl group containing 1 to 6 carbon atoms,
- R₂ represents a halogen atom, a hydrogen atom, an alkoxy group containing 1 to 6 carbon atoms or an alkyl group containing 1 to 6 carbon atoms,
- R₃ represents a halogen atom, a hydrogen atom, an alkoxy group containing 1 to 6 carbon atoms, an alkyl group containing 1 to 6 carbon atoms, an alkylthio group containing 1 to 6 carbon atoms, a cycloalkyl group containing 5 or 6 carbon atoms or a group of formula: 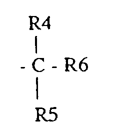 where R4 and R5 represent, independently of one another, a hydrogen atom or an alkyl group containing 1 to 6 carbon atoms and R6 represents a cycloalkyl group containing 3 to 6 carbon atoms, a hydroxymethyl or carboxyl group or alkoxycarbonyl group containing 2 to 7 carbon atoms,
- W represents a carbonyl or hydroxymethylene group, or their salts, the said composition containing polyethylene glycol as solubilizing agent.

2. Composition according to claim 1, characterized in that the active principle is as defined in claim 1, provided that:
- R1 represents a phenyl group
- R2 represents hydrogen
- R3 represents a tert-butyl group
- W represents a carbonyl group.

3. Composition according to claim 1 or 2, characterized in that it contains a weight ratio of polyethylene glycol to compound according to claim 1 of between 100 and 500, and preferably between 100 and 200.

4. Composition according to any one of the preceding claims, characterized in that it contains:
- 0.1 % of compound according to claim 1
- a sufficient quantity of lactic acid to adjust the pH to approximately 4.2
- 16 % of polyethylene glycol
- 60 % of sweetening agent
and the complement of which to 100 % is water.

## Patentansprüche

1. Wäßrige flüssige pharmazeutische Zusammensetzung, frei von oberflächenaktivem Mittel bzw. Tensid, dadurch gekennzeichnet, daß sie als Wirkstoff eine Verbindung entsprechend der Formel 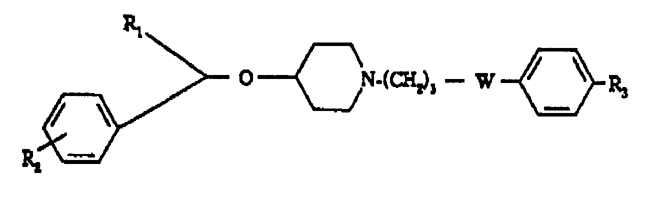 enthält, worin bedeuten:
- R₁ eine Thienylgruppe, eine Phenylgruppe, die gegebenenfalls durch ein Halogen, eine Alkoxygruppe mit 1 bis 6 Kohlenstoffatomen, eine Alkylgruppe mit 1 bis 6 Kohlenstoffatomen, substituiert ist,
- R₂ ein Halogenatom, ein Wasserstoffatom, eine Alkoxygruppe mit 1 bis 6 Kohlenstoffatomen, eine Alkylgruppe mit 1 bis 6 Kohlenstoffatomen,
- R₃ ein Halogenatom, ein Wasserstoffatom, eine Alkoxygruppe mit 1 bis 6 Kohlenstoffatomen, eine Alkylgruppe mit 1 bis 6 Kohlenstoffatomen, eine Alkylthiogruppe mit 1 bis 6 Kohlenstoffatomen, eine Cycloalkylgruppe mit 5 oder 6 Kohlenstoffatomen, oder eine Gruppe der Formel: 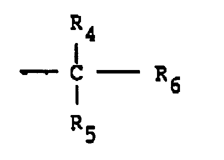 worin R₄ und R₅ unabhängig voneinander ein Wasserstoffatom oder eine Alkylgruppe mit 1 bis 6 Kohlenstoffatomen darstellen, R₆ bedeutet eine Cycloalkylgruppe mit 3 bis 6 Kohlenstoffatomen, Hydroxymethyl, Carboxy oder Alkoxycarbonyl mit 2 bis 7 Kohlenstoffatomen,
- W eine Carbonyl- oder Hydroxymethylengruppe, oder deren Salze, wobei diese Zusammensetzung Polyethylenglykol als solubilisierendes Mittel enthält.

2. Zusammensetzung gemäß Anspruch 1, dadurch gekennzeichnet, daß der Wirkstoff wie in Anspruch 1 definiert ist, vorausgesetzt, daß:
R₁ eine Phenylgruppe,
R₂ Wasserstoff,
R₃ eine tert-Butylgruppe,
W eine Carbonylgruppe
darstellen.

3. Zusammensetzung gemäß Anspruch 1 oder 2, dadurch gekennzeichnet, daß sie ein Gewichtsverhältnis Polyethylenglykol/Verbindung gemäß Anspruch 1 zwischen 100 und 500 und vorzugsweise zwischen 100 und 200 enthält.

4. Zusammensetzung gemäß einem der vorhergehenden Ansprüche, dadurch gekennzeichnet, daß sie enthält:
- 0,1 % Verbindung gemäß Anspruch 1,
- Milchsäure in ausreichender Menge, um das pH bei etwa 4,2 einzustellen,
- 16 % Polyethylenglykol,
- 60 % Süßungsmittel
und deren Ergänzung auf 100 % Wasser ist.
